# EUROPEAN PATENT APPLICATION

(11) **EP 4 544 920 A1**
(43) Date of publication of application: **30.04.2025**
(21) Application number: 24165492.0
(22) Date of filing: 22.03.2024
(51) Int. Cl.: A23L 27/21, A23L 27/24, A23L 27/00, A23L 31/15, C12N 1/16

(54) **METHOD FOR PREPARING, AND INCREASING FLAVOR MOLECULES IN, YEAST EXTRACT AND USE OF THE YEAST EXTRACT**

(30) Priority: 25.10.2023 TW 112140896
(71) Applicant: National Chung-Hsing University, Taichung City 402 (TW)
(72) Inventor: Huang, Chieh-Chen, 402 Taichung City (TW); Lo, Shou-Chen, 300 Hsinchu City (TW); Wu, Dong-Yan, 545 Puli Township (TW)
(74) Representative: Lang, Christian

(57) **Abstract**

A method for preparing, and increasing the flavor molecules in, a yeast extract and a use of the yeast extract are provided. The method includes culturing a yeast strain of the *Kluyveromyces marxianus* species in a yeast culture medium under predetermined conditions in order to increase the diversity and amounts of the sweetness peptides in the yeast culture, thereby providing the yeast extract with strong sweetness and/or umami so that the yeast extract can be used as a food seasoning.

## Description

### BACKGROUND OF THE INVENTION

### 1. Technical Field

The present invention relates to a method for preparing a food ingredient and a use of the food ingredient. More particularly, the invention relates to a method for preparing, and increasing the flavor molecules in, a yeast extract and a use of the yeast extract.

### 2. Description of Related Art

Studies have shown that *Kluyveromyces marxianus* can be commonly found in fermented milk in nature and is a safe food-grade yeast strain having obtained the GRAS and QPS safety certifications in the United States and Europe. Furthermore, *Kluyveromyces marxianus* has such properties as resistance to high temperature and fast growth, can secrete a variety of hydrolytic enzymes and produce ethanol, and has been widely used in food fermentation, alcohol fermentation, and enzyme preparations, among other applications.

Taiwan Patent No. I777730, for example, discloses a method for preparing a yeast peptide and a use of the yeast peptide, and the main technical features of the preparation method consist in culturing *Kluyveromyces marxianus* under specific conditions so as to obtain a yeast peptide product that contains heat shock protein 12. While the yeast peptide product obtained by this prior art technique has bioactivity in many ways (e.g., in promoting the growth of bone and inhibiting oxidation), the preparation method disclosed in the Taiwan patent cannot produce the yeast peptide in a large quantity, and this leads to a prohibitively high production cost when the preparation method is applied to the product end, making it impossible to use the yeast peptide extensively in preparing various products

### BRIEF SUMMARY OF THE INVENTION

The primary objective of the present invention is to provide a method for preparing, and increasing the flavor molecules in, a yeast extract and a use of the yeast extract. The special two-stage preparation process disclosed herein can produce a yeast extract that is highly safe when used in food, has an umami flavor and sweet taste, and can be used in the food processing or food manufacturing industry.

To achieve the foregoing objective, the present invention discloses a method for preparing, and increasing the flavor molecules in, a yeast extract and a use of the yeast extract. The method of the invention for preparing, and increasing the flavor molecules in, a yeast extract uses a two-stage preparation process to produce a yeast extract that is rich in flavor, or has a high level of sweetness and umami to be exact, and because of this, the yeast extract obtained by the disclosed method or an isolate of the yeast extract can be used as food flavor enhancer.

In one embodiment of the present invention, the method disclosed herein for preparing, and increasing the flavor molecules in, a yeast extract includes a fermentation/culture process and an extraction process that are sequentially performed. Both the fermentation process and the extraction process are carried out under predetermined conditions in order to produce a yeast extract that is rich in flavor molecules.

In the fermentation/culture process, the yeast strain used is *Kluyveromyces marxianus,* and the culture medium used is a basic yeast culture medium added with lactic acid at 2-12 %, with the culture temperature being 23-37°C. Given the aforesaid fermentation/culture conditions, a yeast culture can be obtained.

In the extraction process, yeasts are separated from the yeast culture, and using water as the extraction solvent, extraction is performed at an extraction temperature equal to the culture temperature in order to obtain the intended yeast extract. The yeast extract obtained contains flavor molecules that give the yeast extract the activity to provide sweetness and umami.

More specifically, the flavor molecules include at least one sweetness peptide and at least one umami peptide. The sweetness peptide has the sequence of any of SEQ ID NO. 1 to SEQ ID NO. 14 or a homologous sequence bearing at least 95% similarity to any of SEQ ID NO. 1 to SEQ ID NO. 14. The umami peptide has the sequence of any of SEQ ID NO. 15 to SEQ ID NO. 3 5 or a homologous sequence bearing at least 95% similarity to any of SEQ ID NO. 15 to SEQ ID NO. 35.

In one embodiment of the present invention, the yeast bodies are separated and collected from the yeast culture by centrifugation.

In one embodiment of the present invention, both the culture temperature and the extraction temperature are 30 or 37°C, preferably 37°C.

In one embodiment of the present invention, the volume of the extraction solvent is 0.5 to 0.0625 times the volume of the culture medium.

The yeast extract obtained by the foregoing method contains a large amount of sweetness peptides or umami peptides and therefore has a high level of sweetness and umami, meaning the yeast extract disclosed herein can be used as a food flavor enhancer. In addition, any sweetness peptide or umami peptide isolated from the yeast extract or a combination of some or all of the sweetness peptides or umami peptides can be used as a food flavor enhancer for enhancing the sweetness or umami of food.

### BRIEF DESCRIPTION OF THE SEVERAL VIEWS OF THE DRAWINGS

FIG. 1 shows the analysis results of the growth of the *K*. *marxianus* Bot3+7 strain cultured in culture media added with different amounts of lactic acid, with YNLf indicating a YNL culture medium containing food-grade lactic acid.
FIG. 2 shows the analysis result of the protein content of each culture medium in FIG. 1.
FIG. 3 shows the analysis results of the growth of the *K. marxianus* Bot3+7 strain cultured at different temperatures.
FIG. 4 shows the analysis results of the growth of the *K. marxianus* KY3 strain cultured at different temperatures.
FIG. 5 shows the analysis results of the protein contents of extracts obtained by subj ecting the *K. marxianus* Bot3+7 strain cultured at different temperatures to extraction at different temperatures.
FIG. 6 shows the analysis results of the protein contents of extracts obtained by subjecting the *K. marxianus* KY3 strain cultured at different temperatures to extraction at different temperatures.
FIG. 7 shows the analysis results of the protein concentrations of extracts obtained by extracting the *K. marxianus* Bot3+7 strain with different volumes of sterile water.
FIG. 8 shows the analysis results of the net protein contents of yeast extracts obtained by extracting the *K. marxianus* Bot3+7 strain with different volumes of sterile water.
FIG. 9 shows the analysis results of the protein concentrations of yeast extracts cultured under different conditions.
FIG. 10 shows the analysis results of the sweetness, or the equivalent sucrose concentrations, of the yeast extracts in FIG. 9.

### DETAILED DESCRIPTION OF THE INVENTION

The present invention discloses a method for preparing, and increasing the flavor molecules in, a yeast extract and a use of the yeast extract. More specifically, the method disclosed herein for preparing, and increasing the flavor molecules in, a yeast extract involves culturing a yeast strain of the *Kluyveromyces marxianus* species in a yeast culture medium under predetermined conditions in order to increase the diversity and amounts of the sweetness peptides in the yeast culture, thereby providing the yeast extract with strong sweetness and/or umami so that the yeast extract can be used as a food seasoning.

As used herein, the term "*Kluyveromyces marxianus*" refers to a yeast strain of the *Kluyveromyces marxianus* species, such as the *K. marxianus* CCT 7735 strain or any of the *Kluyveromyces marxianus* strains whose deposit numbers are BCRC 20330, 920076, 21477, 21689, 21696, 21422, 21421, 21445, 21698, 20516, 20514, 20540, 21355, 20363, 20438, 21480, 21969, 21965, 21964, 21963, 21499, 21477, 21623, 22115, 22240, 22836, 22026, and 22057.

In one embodiment of the present invention, the method for preparing, and increasing the flavor molecules in, a yeast extract includes the following steps:

Step a: A yeast strain of the *Kluyveromyces marxianus* species is cultured in a yeast culture medium in order to obtain a yeast culture.

The yeast culture medium is a culture medium in which lactic acid is the sole carbon source. More specifically, the yeast culture medium is prepared by adding lactic acid into a basic yeast culture medium until the concentration of lactic acid is 2-12%, such as 4%, 6%, 8%, 7%. 8% , 10%, 12% .

Preferably, the concentration of lactic acid is 4-8%

The culture temperature is 23-37°C, such as 23°C, 25°C, 28°C, 30°C, 32°C, 35°C, 37°C.

Preferably, the culture temperature is 30-37°C.

Step b: Yeasts are separated from the yeast culture.

Step c: The yeasts are mixed with water, which serves as the extraction solvent, broken the yeast cells and then extraction is performed at a predetermined extraction temperature in order to obtain a yeast extract. The predetermined extraction temperature is the same as the culture temperature.

In one embodiment of the present invention, the yeast extract has the greatest amount of flavor molecules when the yeast culture medium contains 4-6% lactic acid.

In one embodiment of the present invention, both the culture temperature and the extraction temperature are 30 or 37°C.

In one embodiment of the present invention, the volume of the extraction solvent is 0.5 to 0.0625 times, preferably 0.0625 times, the volume of the culture medium.

The yeast extract obtained by the foregoing method contains more than 57 proteins. Furthermore, the yeast extract has flavor molecules composed of at least one sweetness peptide and at least one umami peptide that provide the yeast extract with a sweet taste and umami flavor.

More specifically, the sweetness peptide has the sequence of any of SEQ ID NO. 1 to SEQ ID NO. 14 or a homologous sequence bearing at least 95% similarity to any of SEQ ID NO. 1 to SEQ ID NO. 14, and the umami peptide has the sequence of any of SEQ ID NO. 15 to SEQ ID NO. 35 or a homologous sequence bearing at least 95% similarity to any of SEQ ID NO. 15 to SEQ ID NO. 35.

In the yeast extract disclosed herein, the sweetness peptides represented by SEQ ID NO. 2 and SEQ ID NO. 3 are in the greatest amounts. Therefore, it can be inferred that the peptides represented by SEQ ID NO. 2 and SEQ ID NO. 3 are the main sources of sweetness in the yeast extract.

As the yeast extract obtained by the preparation method disclosed herein contains large amounts, and a great variety, of sweetness peptides and umami peptides, not only the yeast extract but also any sweetness peptide isolated from the yeast extract or any combination of the peptides can serve as a food flavor enhancer and be used in the food industry.

The term "basic yeast culture medium" refers to the basic culture medium used in the present invention to culture yeast. The basic yeast culture medium contains elements essential to the growth of yeast but does not contain amino acid. For example, the YNL culture medium is a culture medium that contains lactic acid as the only carbon source. More specifically, the YNL culture medium contains 6.7 g of yeast nitrogen base without amino acids (Difco, Detroit, MI, USA) and 20 g of lactic acid (JT Baker, Philipsburg, NJ, USA) per liter of distilled water.

The term "yeast culture medium" refers to the culture medium used in the present invention to culture yeast. The yeast culture medium contains lactic acid at a predetermined concentration as the only source of carbon for growing yeast.

The term "food flavor enhancer" is also referred to as seasoning, food seasoning, flavoring agent, or flavor enhancing agent. A food flavor enhancer is used mainly to improve or enhance the sweetness and/or umami of food or food products.

The technical features of the present invention and their intended effects are described below with reference to some examples and the accompanying drawings.

The *K. marxianus* Bot3+7 strain used in the following examples was isolated from kerif cultured in Taiwan. Five *Kluyveromyces marxianus* housekeeping gene sequences (namely IPP1, TFC1, GPH1, GSY2, and SGA1) of the isolated *K. marxianus* Bot3+7 strain were amplified by polymerase chain reaction (PCR) according to previous study results and were analyzed at (kmarxianusMLST.ucc.ie). The results show that the *K. marxianus* Bot3+7 strain used in the present invention is highly similar to the *K. marxianus* CCT 7735 strain (which was isolated from Brazilian dairy factory waste water).

The *K. marxianus* KY3 strain used in the following examples has the deposit number BCRC 920076.

The culture time used in the following examples is provided for illustrative purposes only. A person with common general knowledge in the field to which the present invention pertains would be able to adjust the culture time according to such factors as the amount of the yeast being cultured and the volume of the culture medium. The time used in the following examples is not intended to limit the present specification or the scope of the appended claims.

### Example 1: Yeast culture test (1)

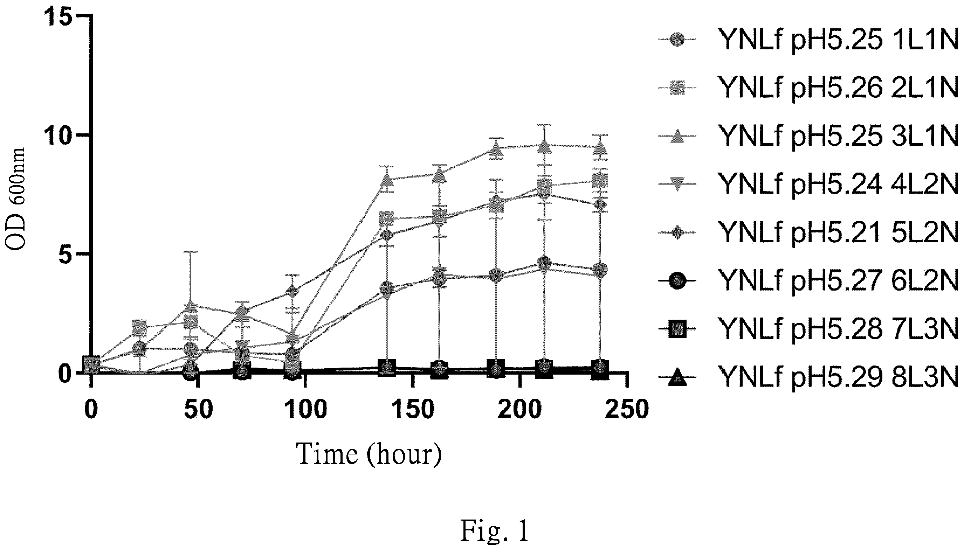

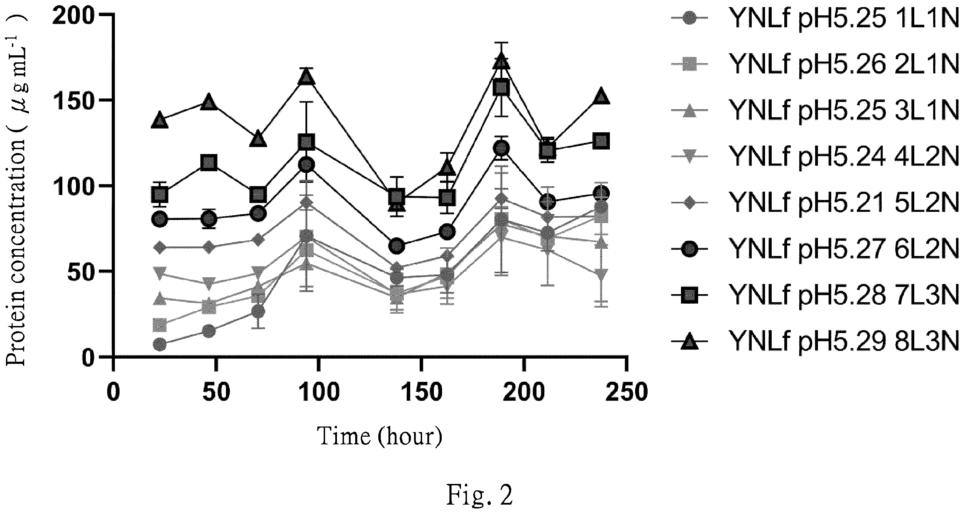

The *K. marxianus* Bot3+7 strain was cultured in a basic yeast culture medium (or more particularly a yeast nitrogen base without amino acids) added with L-lactic acid at 2 wt% (1L1N), 4 wt% (2L1N), 6 wt% (3L1N), 8 wt% (4L2N), 10 wt% (5L2N), 12 wt% (6L2N), 14 wt% (7L3N), or 16 wt% (8L3N), with the temperature of the culture environment being 30°C. The changes in growth of the *K. marxianus* Bot3+7 strain were recorded, and the yeast protein content of each culture medium was analyzed. The results are shown in FIG. 1 and FIG. 2.

It can be known from the results in FIG. 1 that the *K. marxianus* Bot3+7 strain did not grow in those culture media containing 14-16 wt% lactic acid but showed relatively high growth efficiency in the culture medium containing 6 wt% lactic acid. In addition, the results in FIG. 2 show a significant increase in protein content in each of the culture media containing 2-6 wt% lactic acid.

### Example 2: Yeast culture test (2)

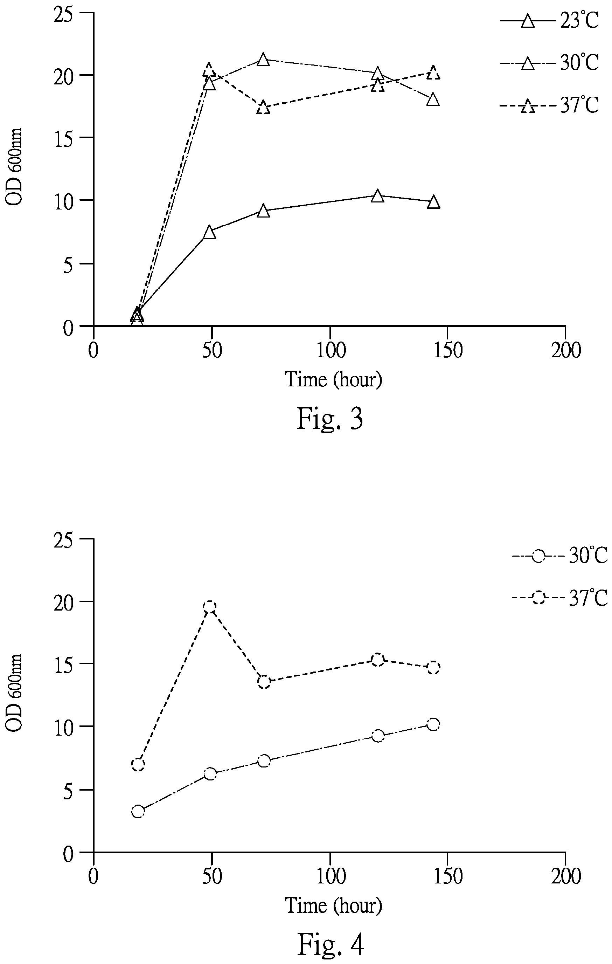

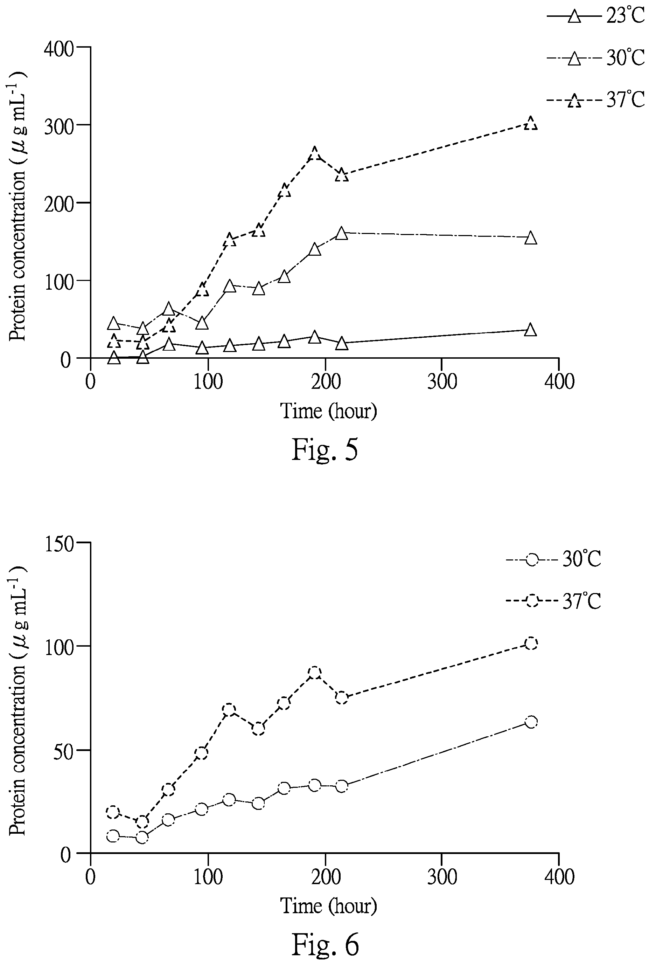

The *K. marxianus* Bot3+7 strain and the *K. marxianus* KY3 strain were each cultured separately at 23°C, 30°C, and 37°C in a basic yeast culture medium (or more particularly a yeast nitrogen base without amino acids) added with L-lactic acid at 6 wt%. The growth of the *K*. *marxianus* Bot3 + 7 strain and *K*. *marxianus* KY3 strain were observed, and the results are shown in FIG. 3 and FIG. 4. Once the culture process was completed, the yeast cultures were centrifuged at 6000 g for five minutes, and the each yeast strain were collected and added with the same amount of sterile water as the corresponding culture medium to suspend the yeast. Following that, yeast was extracted from each suspension at the same temperature as the corresponding culture temperature, and the protein content of each yeast extract obtained was analyzed. The results are shown in FIG. 5 and FIG. 6.

It can be known from the results in FIG. 3 and FIG. 4 that both the *K. marxianus* Bot3+7 strain and the *K. marxianus* KY3 strain grew the least at 23°C, and that each strain grew the most at 30°C or 37°C. In addition, the results in FIG. 5 and FIG. 6 show that the yeast extracts of the *K. marxianus* Bot3+7 strain cultured at different culture temperatures had the highest protein content when the extraction temperature was 37°C, the second highest protein content when the extraction temperature was 30°C, and the lowest protein content when the extraction temperature was 23°C; and that the same is true of the yeast extracts of the *K. marxianus* KY3 strain cultured at different culture temperatures.

### Example 3: Yeast culture test (3)

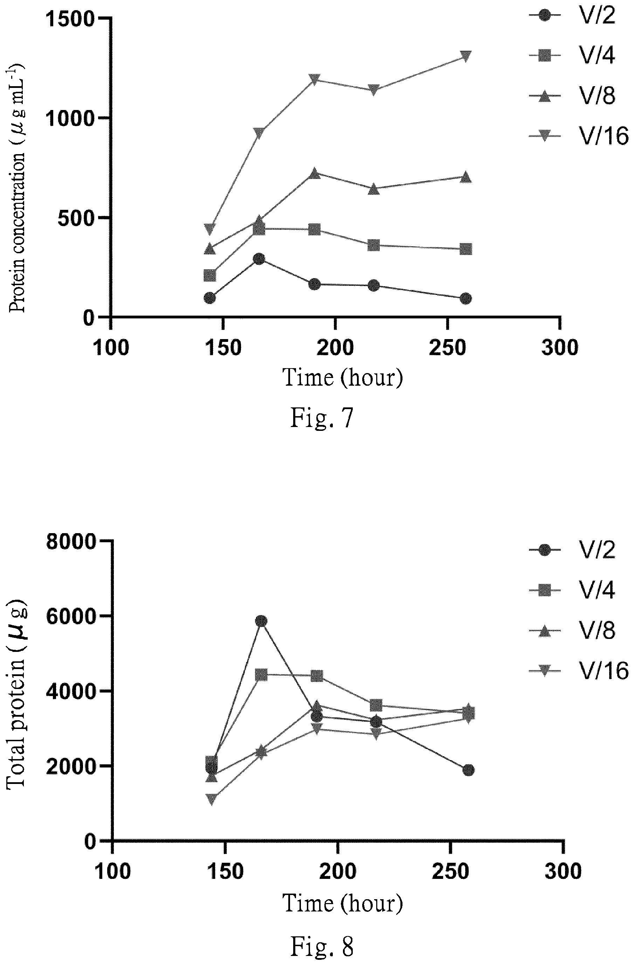

The *K. marxianus* Bot3+7 strain was cultured at 30°C for 186 hours in a basic yeast culture medium (or more particularly a yeast nitrogen base without amino acids) added with L-lactic acid at 2 wt%. Once the culture process was completed, the yeast culture was centrifuged at 6000 g for five minutes, and then yeasts were collected and added with sterile water whose volume was 0.5 times, 0.25 times, 0.125 times, or 0.0625 times the volume of the culture medium to suspend the yeasts. Each suspension was subjected to a yeast extraction process at 30°C, and the protein concentration of each extract obtained was analyzed. The results are shown in FIG. 7. Moreover, the protein concentration of each yeast extract was multiplied by the corresponding volume to determine the net protein content of the yeast extract, and the results are shown in FIG. 8.

It can be known from the results in FIG. 7 that the smaller the volume of the sterile water used to suspend the yeasts, the higher the protein concentration of the yeast extract. However, the results in FIG. 8 indicate that the net protein content of each yeast extract was not markedly different from that of another. It can therefore be inferred that when sterile water is used for yeast extraction, the volume of the sterile water can be as great as, or at least 0.0625 times, the volume of the culture medium.

### Example 4: Analysis of protein composition

The yeast extracts obtained separately in example 3 by extraction with 0.5-times sterile water and 0.25-times sterile water were analyzed by liquid chromatography/tandem mass spectrometry (LC-MS/MS) in order to determine their protein compositions, and the top 100 proteins (based on their respective contents determined by the analysis) in the yeast extract obtained by extraction with 0.25-times sterile water were compared against the proteins identified in the yeast extract obtained by extraction with 0.5-times sterile water. The comparison reveals that both yeast extracts contained a total of 57 major proteins in common, as listed in Table 1 below.

**Table 1: Intersection set obtained by comparison between proteins in two yeast extracts**

| UniProt Entry Name | Protein Name |
|---|---|
| W0T7K9_KLUMD | Phosphopyruvate hydratase (EC 4.2.1.11) |
| W0TDE9_KLUMD | Triosephosphate isomerase (EC 5.3.1.1) |
| W0TJU9_KLUMD | Histone H4 |
| TPIS_KLUMA | Triosephosphate isomerase (EC 5.3.1.1) |
| W0TFW6_KLUMD | Dihydrolipoyl dehydrogenase (EC 1.8.1.4) |
| W0T3H5_KLUMD | Phosphoglycerate kinase (EC 2.7.2.3) |
| W0TBU7_KLUMD | Cytochrome c |
| W0T992_KLUMD | Peroxiredoxin type-2 |
| W0TCV6_KLUMD | Transaldolase (EC 2.2.1.2) |
| W0T5P3_KLUMD | Heat shock protein 60 |
| W0TFX4_KLUMD | NADPH-dependent methylglyoxal reductase GRE2 |
| A4Z4U8_KLUMA | Pyrophosphate phospho-hydrolase (EC 3.6.1.1) |
| G3P1_KLUMA | Glyceraldehyde-3-phosphate dehydrogenase 1 (GAPDH 1) (EC 1.2.1.12) |
| W0T4T1_KLUMD | Histone H2B |
| W0THL5_KLUMD | Probable 2-methylcitrate dehydratase |
| W0TC44_KLUMD | Peroxiredoxin TSA1 |
| W0TF55_KLUMD | Phosphotransferase (EC 2.7.1.-) |
| W0TCK6_KLUMD | PPIase (Peptidyl-prolyl cis-trans isomerase) (EC 5.2.1.8) |
| W0TCX8_KLUMD | Potassium-activated aldehyde dehydrogenase |
| W0T9E2_KLUMD | Alpha-1,4 glucan phosphorylase (EC 2.4.1.1) |
| W0TKF9_KLUMD | 5-methyltetrahydropteroyltriglutamate--homocysteine S-methyltransferase (EC 2.1.1.14) |
| W0TD37_KLUMD | Pyridoxal 5'-phosphate synthase (glutamine hydrolyzing) (EC 4.3.3.6) |
| W0TAV6_KLUMD | Mitochondrial ketol-acid reductoisomerase (Acetohydroxy-acid reductoisomerase) (Alpha-keto-beta-hydroxylacyl reductoisomerase) (EC 1.1.1.86) |
| W0T8K0_KLUMD | Glutathione reductase (EC 1.8.1.7) |
| W0T4R5_KLUMD | Glucose-6-phosphate isomerase (EC 5.3.1.9) |
| W0T598_KLUMD | BMH2 protein |
| W0TFM6_KLUMD | Non-histone chromosomal protein 6 |
| W0T947_KLUMD | Putative reductase 1 |
| W0THE4_KLUMD | Malate synthase (EC 2.3.3.9) |
| W0TEG9_KLUMD | Malate dehydrogenase (EC 1.1.1.37) |
| PDC1_KLUMA | Pyruvate decarboxylase (EC 4.1.1.1) |
| W0T2L5_KLUMD | Citrulline-aspartate ligase (EC 6.3.4.5) |
| W0T3P9_KLUMD | Phosphotransferase (EC 2.7.1.-) |
| W0TI19_KLUMD | HIT family protein 1 |
| W0T626_KLUMD | Profilin |
| W0TGG7_KLUMD | Isocitric dehydrogenase (NAD(+)-specific ICDH) (EC 1.1.1.41) |
| W0T9W3_KLUMD | FBP aldolase (EC 4.1.2.13) |
| W0T9G9_KLUMD | Aminopeptidase (EC 3.4.11.-) |
| W0TC93_KLUMD | Alcohol dehydrogenase (EC 1.1.1.1) |
| W0TBT9_KLUMD | Fumarate reductase (EC 1.3.1.6) |
| W0TAV4_KLUMD | 12-kDa heat shock protein |
| W0TJJ1_KLUMD | Sphingolipid long chain base-responsive protein LSP1 |
| W0TDE0_KLUMD | Proteasome component C7-alpha |
| W0TEV0_KLUMD | UTP--glucose-1-phosphate uridylyltransferase (EC 2.7.7.9) |
| W0T8N1_KLUMD | Adenosylhomocysteinase (EC 3.13.2.1) |
| W0TD70_KLUMD | ATP-dependent molecular chaperone HSC82 |
| W0TB47_KLUMD | Omithine aminotransferase (EC 2.6.1.13) |
| W0THY0_KLUMD | Heat shock protein SSA3 |
| D1LES1_KLUMA | Methylisocitrate lyase (EC 4.1.3.30) |
| W0T7K6_KLUMD | Mitochondrial aconitate hydratase (Aconitase) (EC 4.2.1.-) |
| W0TFA8_KLUMD | Citrate synthase |
| W0T9B9_KLUMD | Gamma-amino-N-butyrate transaminase (GABA aminotransferase) (EC 2.6.1.19) |
| W0TF96_KLUMD | Fructose-bisphosphatase (EC 3.1.3.11) |
| W0THR5_KLUMD | Transketolase (EC 2.2.1.1) |
| Q617B7_KLUMA | Old yellow enzyme |
| W0TFP1_KLUMD | Peroxidase (EC 1.11.1.-) |
| W0T939_KLUMD | Heat shock protein 26 |

It can be known from the results in Table 1 that, regardless of the volume of the sterile water used for extraction, all the liquid yeast extracts contained peptides having a sweet taste and/or umami flavor, such as 12-kDa heat shock protein and phosphopyruvate hydratase. In other words, the yeast culture cultured by the method of the present invention or an extract thereof has a sweet taste and/or umami flavor.

### Example 5: Yeast culture test (4)

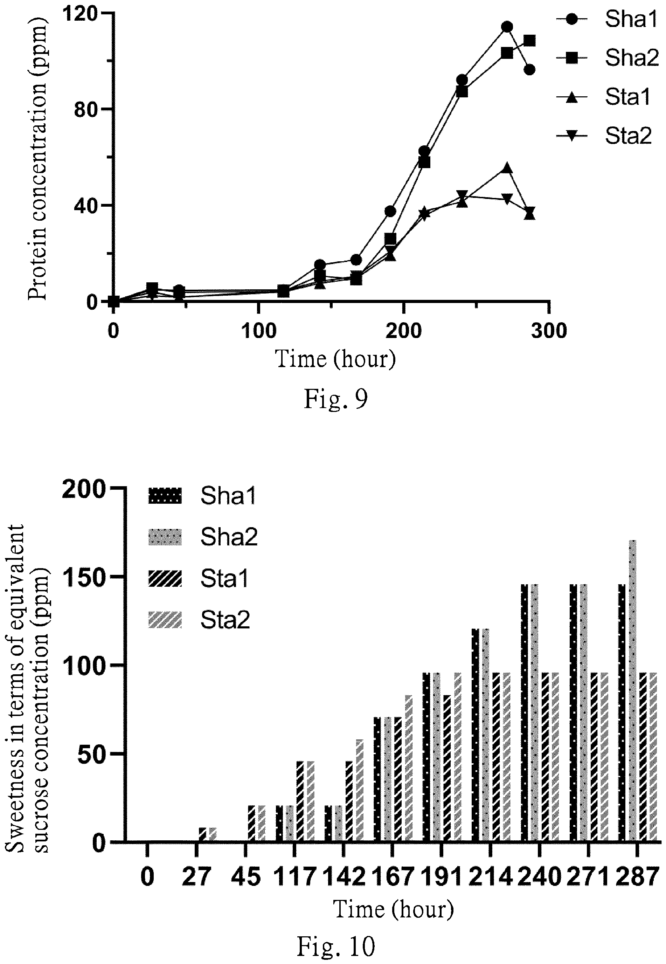

The *K. marxianus* Bot3+7 strain was cultured under the conditions of 30°C and 150 rpm for 14.5 hours in a basic yeast culture medium (or more particularly a yeast nitrogen base without amino acids) added with L-lactic acid at 6 wt% (the total volume of the yeast culture medium being 500 mL). Once the culture process was completed, the yeast culture was centrifuged at 4800 g and 4°C for ten minutes, and then yeasts were collected, added with the same volume of sterile water as the culture medium, and then either shaken at 150 rpm or allowed to rest in a 30°C environment. The protein concentrations of the resulting yeast extracts are shown in FIG. 9, in which: each Sha group involved shaking the corresponding yeast suspension at 150 rpm in a 30°C environment for the first 45 hours of the extraction process and then allowing the suspension to rest in the 30°C environment for the rest of the extraction process, and each Sta group involved allowing the corresponding yeast suspension to rest in a 30°C environment for the entire extraction process. Each time the protein concentration of a yeast extract sample was measured, the taste of the sample was also tested and compared against the sweetness levels of aqueous sucrose solutions of different concentrations. The results are shown in FIG. 10.

It can be known from the results in FIG. 9 and FIG. 10 that the sweet taste (sweetness) of each yeast extract increased with the protein concentration of the yeast extract.

### Example 6: Yeast culture test (5)

Referring to the contents of example 1, the *K. marxianus* Bot3+7 strain was cultured in a culture medium containing 2 wt%, 4 wt%, 6 wt%, 8 wt%, or 10 wt% L-lactic acid. Once the culture process was completed, the yeast cultures were subjected to extraction with water by the resting approach in example 5. The yeast extract obtained in each group was then analyzed by LC-MS/MS in order to identify whole-protein segments and peptides. Peptide sequences with a molecular weight less than 1.5 kDa were extracted from the LC-MS/MS analysis results, and the extraction results were compared against a peptide database (the BIOPEP-UWM database, https://biochemia.uwm.edu.pl/biopep-uwm/) and were statistically analyzed in order to determine the frequencies of the occurrence of sweetness-related active sites and umami-related active sites in each peptide. A peptide was determined to have sweetness or umami when it had a relatively high frequency of occurrence of sweetness-related active sites or umami-related active sites.

According to the analysis results of the frequencies of occurrence of sweetness-related active sites and umami-related active sites, the yeast extract disclosed herein contains 14 peptides (i.e., the peptides represented by SEQ ID NO. 1 to SEQ ID NO. 14) in each of which the frequency of occurrence of sweetness-related active sites is greater than 0.5, as shown in Table 2 below, and the 14 peptides make up 42.4% of those peptides with a molecular weight less than 1.5 kDa. Moreover, the yeast extract disclosed herein contains 21 peptides (i.e., the peptides represented by SEQ ID NO. 15 to SEQ ID NO. 35) in each of which the frequency of occurrence of umami-related active sites is greater than 0.5, as shown in Table 3 below, and the 21 peptides make up 30.1% of the peptides with a molecular weight less than 1.5 kDa. The foregoing results indicate that the yeast extract disclosed herein can be expected to have the activity to provide both sweetness and umami.

According to literature (Xiaolan Bao et al., Sensory and structural characterization of umami peptides derived from sunflower seed (Caracterización sensorial y estructural de los péptidos umami derivados de la semilla de girasol), CyTA - Journal of Food, Volume 18, 2020 - Issue 1, pages 485-492), peptide molecules that provide gustatory sensation should be lighter than 3 kDa, and it can be known from the results in Table 2 that all the peptides represented by SEQ ID NO. 1 to SEQ ID NO. 14 have molecular weights less than 1.5 kDa and include sweetness-related active sites whose frequency of occurrence is higher than 50%. That is to say, the yeast extract disclosed herein can effectively provide sweetness or umami and serve as a food seasoning because it contains the peptides represented by SEQ ID NO. 1 to SEQ ID NO. 14.

It can also be known from the results in Table 2 that, of all the lighter-than-1.5 kDa peptides in the yeast extract disclosed herein, the peptides represented by SEQ ID NO. 2 and SEQ ID NO. 3 have the highest percentages (13.2% and 8.4% respectively). This means that the main sources of sweetness of the yeast extract disclosed herein should be the peptides represented by SEQ ID NO. 2 and SEQ ID NO. 3.

**Table 2. Peptides in which the frequency of occurrence of sweetness-related active sites is higher than 0.5**

| No. of amino acid sequence | Peptide sequence | Count | Percentage in the group of peptides lighter than 1.5 kDa | Frequency of occurrence of sweetness-related active sites (A) |
|---|---|---|---|---|
| SEQ ID NO.:1 | PPPPPPPPP | 2 | 1.886792 | 1 |
| SEQ ID NO.:2 | GPPPPPPPPP | 14 | 13.20755 | 1 |
| SEQ ID NO.:3 | GPPPPPPPPPA | 9 | 8.490566 | 1 |
| SEQ ID NO.:4 | DVPVVPGTPGP | 1 | 0.943396 | 0.8182 |
| SEQ ID NO.:5 | DIPVPKP | 2 | 1.886792 | 0.7143 |
| SEQ ID NO.:6 | DLPVPKP | 1 | 0.943396 | 0.7143 |
| SEQ ID NO.:7 | VGDDVAVGR | 1 | 0.943396 | 0.6667 |
| SEQ ID NO.:8 | DLQNPPPPP | 4 | 3.773585 | 0.5556 |
| SEQ ID NO.:9 | KGDWPLDTK | 1 | 0.943396 | 0.5556 |
| SEQ ID NO.:10 | DLQNPPPPPS | 3 | 2.830189 | 0.5 |
| SEQ ID NO.:11 | IIAEPWTIVGGK | 1 | 0.943396 | 0.5 |
| SEQ ID NO.:12 | DTKLPLVG | 4 | 3.773585 | 0.5 |
| SEQ ID NO.:13 | VDEPAVRE | 1 | 0.943396 | 0.5 |
| SEQ ID NO :14 | GLDPPASPDDPTPE | 1 | 0.943396 | 0.5 |
| | Total | 45 | 42.45283 | |

**Table 3. Peptides in which the frequency of occurrence of umami-related active sites is higher than 0.5**

| No. of amino acid sequence | Peptide sequence | Count | Percentage in the group of peptides lighter than 1.5 kDa | Frequency of occurrence of umami-related active sites (A) |
|---|---|---|---|---|
| SEQ ID NO :15 | EDDVVKL | 1 | 0.943396226 | 1 |
| SEQ ID NO :16 | LDDELKPT | 1 | 0.943396226 | 1 |
| SEQ ID NO :17 | FDDLIEE | 2 | 1.886792453 | 0.8571 |
| SEQ ID NO :18 | MELITEE | 2 | 1.886792453 | 0.8571 |
| SEQ ID NO :19 | IEEEGIALR | 1 | 0.943396226 | 0.7778 |
| SEQ ID NO :20 | LGDDIEDRE | 2 | 1.886792453 | 0.7778 |
| SEQ ID NO :21 | IDDDITRM | 1 | 0.943396226 | 0.75 |
| SEQ ID NO :22 | IDDEIVKT | 1 | 0.943396226 | 0.75 |
| SEQ ID NO :23 | LDDELKPTKPS | 1 | 0.943396226 | 0.7273 |
| SEQ ID NO :24 | DEGKDIDIPNLVE | 1 | 0.943396226 | 0.6923 |
| SEQ ID NO :25 | VDEPAVRE | 1 | 0.943396226 | 0.625 |
| SEQ ID NO :26 | VDDIIDRLLE | 2 | 1.886792453 | 0.6 |
| SEQ ID NO :27 | ENDAPIVEPVFE | 1 | 0.943396226 | 0.5833 |
| SEQ ID NO :28 | DDGTLKL | 3 | 2.830188679 | 0.5741 |
| SEQ ID NO :29 | DDGTIKI | 3 | 2.830188679 | 0.5714 |
| SEQ ID NO :30 | VGDDVAVGR | 1 | 0.943396226 | 0.5556 |
| SEQ ID NO :31 | NDAPIVEPVFE | 2 | 1.886792453 | 0.5455 |
| SEQ ID NO :32 | DDGTLKLN | 1 | 0.943396226 | 0.5 |
| SEQ ID NO :33 | IGEPIDER | 3 | 2.830188679 | 0.5 |
| SEQ ID NO :34 | GGDEGRLF | 1 | 0.943396226 | 0.5 |
| SEQ ID NO :35 | LPDEPIDK | 1 | 0.943396226 | 0.5 |
| | Total | 32 | 30.18867925 | |

## Claims

1. A method for preparing, and increasing flavor molecules in, a yeast extract, comprising:
step a: culturing a yeast of the *Kluyveromyces marxianus* species in a yeast culture medium at a predetermined culture temperature to produce a yeast culture, wherein the yeast culture medium is a basic yeast culture medium containing 2-12% lactic acid, with the lactic acid being a sole carbon source in the yeast culture medium, and the predetermined culture temperature is 23-37°C;
step b: separating yeasts from the yeast culture; and
step c: mixing the yeasts with water serving as an extraction solvent, and performing extraction at a predetermined extraction temperature to obtain the yeast extract, wherein the predetermined extraction temperature is equal to the predetermined culture temperature.

2. The method for preparing, and increasing flavor molecules in, a yeast extract as claimed in claim 1, wherein the lactic acid in the step a has a concentration of 4-6%.

3. The method for preparing, and increasing flavor molecules in, a yeast extract as claimed in claim 1, wherein the extraction solvent in the step c has a volume 0.5 to 0.0625 times as great as a volume of the yeast culture medium.

4. The method for preparing, and increasing flavor molecules in, a yeast extract as claimed in claim 1, wherein both the predetermined culture temperature in the step a and the predetermined extraction temperature in the step c are 37°C.

5. The method for preparing, and increasing flavor molecules in, a yeast extract as claimed in claim 1, wherein in the step b, the yeasts are separated from the yeast culture by centrifugation.

6. A yeast extract prepared by the method of any of claims 1 to 5, comprising peptides represented by SEQ ID NO. 1 to SEQ ID NO. 35.

7. A use of flavor molecules in preparing a food flavor enhancer, wherein the flavor molecules comprise at least one peptide selected from the group consisting of peptides represented by SEQ ID NO. 1 to SEQ ID NO. 35.

8. The use of flavor molecules in preparing a food flavor enhancer as claimed in claim 7, wherein the flavor molecules are used to provide sweetness and comprise the peptides represented by SEQ ID NO. 1 to SEQ ID NO. 14.

9. The use of flavor molecules in preparing a food flavor enhancer as claimed in claim 7, wherein the flavor molecules are used to provide umami and comprise the peptides represented by SEQ ID NO. 15 to SEQ ID NO. 3 5.

10. The use of flavor molecules in preparing a food flavor enhancer as claimed in claim 7, wherein the flavor molecules are a yeast extract or an isolate thereof, and the yeast extract is prepared by the method of any of claims 1 to 5.
